(19) **Europäisches Patentamt / European Patent Office / Office européen des brevets**

(11) **EP 4 783 187 A1**

(12) # EUROPEAN PATENT APPLICATION

(43) Date of publication:
**29.07.2026 Bulletin 2026/31**

(21) Application number: **26154338.3**

(22) Date of filing: **27.01.2026**

(51) International Patent Classification (IPC):
**G16H 30/40** (2018.01)  **A61B 5/00** (2006.01)
**G06F 18/20** (2023.01)  **G06N 3/02** (2006.01)
**G06T 7/00** (2017.01)  **G16H 40/63** (2018.01)
**G16H 50/20** (2018.01)  **G16H 50/70** (2018.01)

(52) Cooperative Patent Classification (CPC):
**G16H 50/20; A61B 5/0077; A61B 5/015;
A61B 5/445; A61B 5/7267; G06N 3/02;
G06N 3/045; G06N 3/08; G06T 7/0012;
G06T 7/0016; G06V 10/143; G06V 10/454;
G06V 10/764; G06V 10/806; G06V 10/82;** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **28.01.2025 IT 202500001494**

(71) Applicant: **Politecnico Di Torino
10129 Torino (IT)**

(72) Inventors:
• **Molinari, Filippo
10129 Torino (IT)**
• **Secco, Jacopo
10129 Torino (IT)**
• **Becchi, Sara
10129 Torino (IT)**
• **Cavazzana, Rosanna
10129 Torino (IT)**

(74) Representative: **Mola, Edoardo et al
Praxi Intellectual Property S.p.A.
Corso Vittorio Emanuele II, 3
10125 Torino (IT)**

(54) **SYSTEM AND COMPUTER-IMPLEMENTED METHOD FOR MULTIMODAL IMAGING AND MACHINE-LEARNING-BASED DIAGNOSIS OF WOUND INFECTIONS**

(57) The invention relates to a system and a computer-implemented method for identifying wound infections using multimodal images and machine learning. The system integrates data from RGB and thermal images, processes them using trained algorithms to detect wound boundaries and temperature gradients, and identifies potential infections. The system provides therapeutic recommendations by comparing patient data with a structured database of known infection cases.

**FIG. 1**

EP 4 783 187 A1

(52) Cooperative Patent Classification (CPC): (Cont.)
**G16H 30/40; G16H 40/63; G16H 50/70;**
G06T 2207/10024; G06T 2207/10048;
G06T 2207/20084; G06T 2207/30088;
G06V 2201/03

**Description**

TECHNICAL FIELD

The technical field of the present invention relates to imaging and medical diagnostic systems, in particular those exploiting computer-implemented methods and machine learning for the analysis of wound conditions. This concerns the use of multimodal imaging techniques (RGB and thermography) combined with algorithmic processing to identify and analyse infectious states of wounds, providing therapeutic recommendations based on a structured database of known cases.

STATE OF THE ART

Approximately 2% of the world population suffers from skin ulcers, which are skin wounds caused by various pathologies, such as diabetes, heart disease, post-surgical infections, and pressure sores. The most affected individuals are mainly elderly people (over 65 years of age), who are more vulnerable to systemic physiological imbalances.

The treatment of these wounds is based on two approaches:

> 1. Continuous monitoring of the wound, in order to track its evolution both from a clinical and a morphological point of view.
> 2. Study of the pathology that caused the ulcer and personalised treatment based on the characteristics of the patient.

In wound monitoring, devices are used to measure the ulcer so as to add updated data on its condition. However, as regards the treatment of the underlying pathology, there is still a strong reliance on the experience of operators, since advanced and patient-targeted technologies are not available on the market.

A further problem is the analysis of wound infections. Current methods do not provide exhaustive information on bacterial or viral proliferation in a non-invasive manner. The most common techniques are swabbing or biopsy of the wound tissue, which entail high costs, procedural complications, and pain for the patient.

At present, when errors are made in the evaluation and treatment of ulcers, the consequences can be severe: in 70% of cases the wound does not heal, in 32% of cases limb amputation occurs, and in 17% of cases the patient dies within the first two years from the onset of the ulcer.

There is therefore a strongly felt need for a non-invasive technology capable of analysing the infectious state of a wound and of recommending a treatment and a dosage tailored to the patient.

OBJECTS AND SUMMARY OF THE INVENTION

The object of the present computer-implemented invention is a method for identifying infectious states of a wound, comprising the steps of:

- Receiving a first image, RGB, of the wound captured using an RGB camera,
- Receiving a second image, thermographic, of the same wound captured using a thermal camera aligned with the camera, such that the second image shares with the first image the same reference system, where by the same reference system it is meant that the region captured by the two images is the same,
- Processing the first image with first identification means, preferably a trained machine learning algorithm, of a contour of the wound based on an analysis of a texture variation in the first image,
- Processing the second image with second identification means, preferably a second trained machine learning algorithm operating in parallel with the first identification means, of regions with a temperature gradient exceeding a predetermined threshold,
- Selecting the regions falling within or on the contour, namely the regions that present both a significant texture according to the first means and a significant temperature gradient for the second means, where by significant it is meant greater than an internal threshold of the identification means identified during the training phase,
- Processing said selected regions by means of third identification means configured to compare said regions with images classified according to a respective value of a parameter indicating an infectious stage, that is, the third network determines whether, on the basis of the identification by the first two means, an infectious state is identified,
- Acquiring characteristic data of the patient,
- Composing an input data set containing said value of said parameter and said characteristic data of the patient; that is, a data set is generated comprising both information relating to the infectious state of the wound identified by the first, second and third identification means and the characteristic data of the patient, such that the patient is associated with their own wound,
- Selecting an output data set from a plurality of data sets previously stored in a database, by means of a likelihood algorithm, wherein a respective therapy is associated with each of said previously stored data sets; that is, the previously composed data set is used as a search key in a database containing a plurality of data sets comprising information on the wound and the patient associated therewith; in this phase, the most similar patient is searched for in the database both in terms of patient characteristic data and wound data with respect to the input data set,

- Indicating the therapy associated with the output data set.

Another object of the present invention is a device configured to carry out the method previously described.

These and other aspects of the invention will be better described in the detailed description and in the claims.

BRIEF DESCRIPTION OF THE DRAWINGS

Preferred embodiments of the present invention will be described below purely by way of example, with reference to the accompanying drawings, in which:

Figure 1. Schematic representation of the present invention.
Figure 2. Schematic representation of algorithm 1, in which the blocks and nodes of the neural networks are highlighted.
Figure 3. Schematic representation of the micro- and macro-structure of algorithm 2.
Figure 4a. Validation of the RGB network of algorithm 1, in which the non-granular areas of the wound are highlighted.
Figure 4b. Validation of the thermographic network of algorithm 1, in which the areas with a high temperature gradient are highlighted.
Figure 4c. Output of algorithm 1, in which images and information from the RGB and thermographic networks are superimposed.
Figure 5. Graphical representation of the validation of algorithm 2, in which a transition is made from an initial patient treatment policy to an optimal policy.

DETAILED DESCRIPTION OF THE INVENTION

The present invention relates to a device for the identification and progression of infectious states of a cutaneous ulcer wound. This is achieved through the synergistic use of an RGB digital camera, an infrared thermal camera, and two distinct neural networks suitably trained for the recognition of the infectious state and for the prediction of the progression thereof with the related therapy. Each component will be described in detail in the following paragraphs.

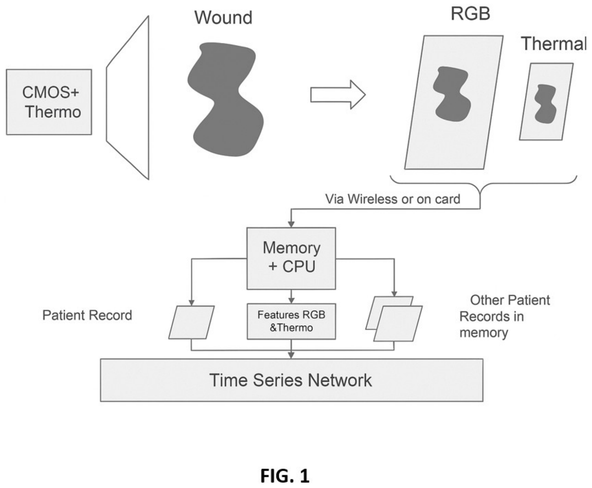

First, however, a flow diagram according to the present invention is described, shown in Figure 1. With the RGB camera and the thermal camera, two images of the area of interest on the patient's body are simultaneously acquired. It is important to underline that the RGB camera and the thermal camera must capture the same subject, that is, their focal points and their horizons must be aligned, or in other words the RGB camera and the thermal camera share the same reference system. This is evident because in the subsequent step the two acquired images are superimposed and provided as input to a processing unit and memory means, on which the two algorithms according to the present invention are present. The first algorithm receives as input the pair of images and provides as output the identification and classification of an infection. In the event that the infection is present, the output of the first algorithm is provided as input to the second algorithm, which compares the input with a plurality of known cases, loaded in the memory unit, of ulcer infections and recommends the best treatment on the basis of the progression of the known cases; simultaneously, the input is inserted into a folder of known cases in order to expand the library of cases of the second algorithm.

Briefly, the present invention acquires at least one RGB image and one thermographic image and, from these, determines the presence of an infectious state and, if this is present, the recommended therapy for treating said infectious state.

It is important to note that in the context of the present invention, the term "identify" refers to the process of detecting, recognising and characterising specific attributes or characteristics of the lesion using computational techniques. In particular, in the context of convolutional neural networks (CNNs), the term includes the extraction and classification of features from input data (for example, images or sensor signals) based on predefined criteria or criteria learned by the model. This process is based on the layers of the neural network that process the data pixel by pixel (or sensor point by sensor point) in a spatially localised manner, where each node of the network interacts with its neighbours to extract high-level features such as wound edges, colour variations or texture characteristics indicative of the condition of the wound.

The RGB camera

A camera equipped with CMOS or CCD sensors capable of acquiring RGB images, that is, a camera that uses visible light, namely luminous radiation with a wavelength comprised between 400 and 700 nm. The camera has no upper limit with regard to resolution, but has a lower limit; in particular, the camera must be able to capture a sufficient level of detail to identify the wound. According to the present invention, this resolution limit is 5 Mpx.

The thermal camera

The thermal camera or thermal imaging camera has no particular limitations, but must acquire images in the infrared, that is, luminous radiation having a wavelength comprised between 2 and 14 $\mu$m. As for the RGB camera, the resolution has no upper limit, while the lower limit is 30 pixels $\times$ 40 pixels.

It should be underlined that the thermal camera and the RGB camera do not necessarily have to have the same resolution; in fact, it is possible to scale the image dimensions in such a way that there is a direct correspondence between the pixels of the RGB image and the thermographic image. Indeed, it is important that said images are aligned and superimposable. This is achieved synergistically, by acquiring the images while taking care to align the focal points and horizons of the RGB camera and the thermal camera and by scaling the dimensions of the images so that the two images have the same size, that is, the same number of pixels, and in which each pixel contains the same information (that is, they frame corresponding areas).

Algorithm 1

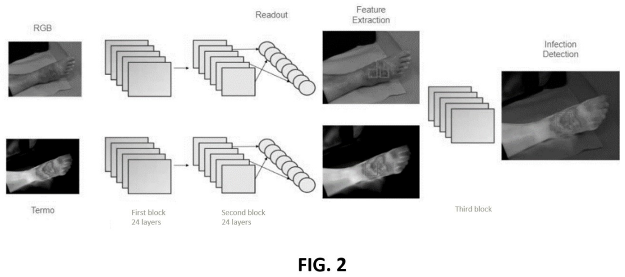

The first algorithm receives as input the RGB image and the thermographic image and provides as output the identification of an infectious state. It should be noted that the algorithm does not require data obtained through specific sensors, but it is sufficient to receive at least one RGB image and one thermographic image. The architecture of algorithm 1, which is an algorithm of the Cellular Neural Network (CNN) type, is shown in Figure 2. It comprises two parallel networks that converge into a final single network, that is, there are three networks in total. The two parallel networks have an identical structure and operate one on the RGB image and the other on the thermographic image. The networks are composed as follows: three blocks, in which the first two blocks are identical and formed by 24 layers comprising a variable number of nodes ranging from 208 up to 1024, while the third block is different and has no layers, but only 24 nodes. The term block is herein defined as: a plurality of layers; the term layer is herein defined as: a plurality of nodes; the term node is herein defined as: the unit where an operation is performed, e.g. an addition.

Each block has a specific purpose. In particular, the first block receives as input an RGB or thermographic image and subdivides it into 24 quadrants of identical size; the second block receives as input the RGB or thermographic image subdivided into 24 quadrants and subdivides said 24 quadrants into a further 24 quadrants; the third block receives as input the image subdivided into 24 quadrants, in which each quadrant is divided into a further 24 quadrants, and transmits it to the third neural network. The third neural network, referred to as the union network, receives as input the outputs of the RGB and thermographic neural networks and analyses the quadrants of the images that are considered relevant by the RGB and thermographic neural networks. At the output of the third neural network, an image representative of the infectious state is obtained. In fact, by means of the RGB neural network, the granulometry of the wound is estimated; in particular, the areas of the wound in the image are classified as granular or non-granular, the latter corresponding to the wound bed or necrotic tissue or fibrin; while by means of the thermographic network, those areas with high temperature gradients potentially representative of the presence of an infection are identified. The combination of granulometric and thermal information allows the union network to evaluate the infectious state of the wound. It should be underlined that the third network has an active role in the identification, since the presence of granulometry and a temperature gradient are not always indicative of an infectious state. In fact, the third network is trained to recognise an infectious state starting from granulometric and temperature gradient information; without this training it would not be possible to obtain identification of the infectious state using solely the recognition carried out by the RGB network and the thermographic network.

It is known that for neural networks the training phase is of fundamental importance. According to the present invention, training was carried out using a series of RGB and thermographic images of cutaneous ulcers, in which the two cameras were aligned as described previously, independently of the actual clinical state of the ulcer. The thermographic images were then subsequently processed in order to obtain the spatial derivative image thereof; in this way a greyscale image is obtained in which white indicates the area with the highest temperature and black indicates the area with the lowest temperature. On each of these images, bounding boxes were drawn, that is, the areas deemed to be of interest were graphically highlighted manually by experts in the field. Accordingly, in the RGB images the non-granular areas of the wound were highlighted with bounding boxes, while in the thermal images the areas where there was a significant temperature gradient were highlighted, where by significant it is meant a value that the network itself determines as significant on the basis of training for the purpose of achieving the desired result; such value varies during training but is then fixed once the network is trained. The images with bounding boxes representative of the areas of interest, i.e. non-granular areas and areas with a significant temperature gradient, are provided to the union algorithm. Schematically, the training process is as follows:

1. Splitting of the image

Each image is divided into at least 24 quadrants.

Each pixel in a quadrant is connected to a node in the neural network, either RGB or thermographic.

For each node, a value "o" is calculated using the formula:

$o = \sum_{i,j=[-1,0,1]} x_{i,j} u_{i,j} - \theta,$ in which x corresponds to the internal value of the computational node at position i,j, while u corresponds to the value of the input, which may be 0 or 1 depending on whether the pixel is outside or not of the bounding box, $\theta$ is the threshold value. If the sum exceeds the threshold, the node is activated.

2. Further splitting into quadrants

Each quadrant is further subdivided into at least 24 sub-quadrants.

If the sub-quadrants have a sum of values above a certain threshold, the calculation performed in the first step is repeated, obtaining a new value $o_2$.

3. Subsequent steps

The $o_2$ values of the sub-quadrants are summed. If the sum exceeds a threshold, the node associated with the third level of the network is activated with value $o_3$, whose value is either 1 or 0.

4. Superposition of the networks

If $o_3$ is equal to 1, the layer of the network associated with that node is taken and superimposed on the corresponding layer of the other network, that is, the RGB network is superimposed on the thermographic network.

This superposition generates a new image, and the same process of subdivision and calculation is repeated on the new superimposed nodes.

5. Feedback

After each cycle, there is a feedback mechanism that updates the nodes based on what has been learned, using the following formula that also takes into account the previous iteration:

$$dx/dt = x(t-1) + o_3 (2u - o_3)$$

In summary, this process subdivides the image into quadrants, performs calculations and, based on threshold values, activates or does not activate the nodes, and finally, thanks to the training and feedback cycles, the model learns to correctly recognise the important areas, i.e. non-granular areas and areas with a significant temperature gradient, of the RGB and thermographic images. At a practical level, once the model is trained, the nodes no longer change during use. That is, when the model is used on new images it follows exactly the same steps described, but the node values are fixed.

Finally, the union network, after superimposing the images, identifies the infectious state. This is again achieved through training, in which experts in the field have identified the infectious state and, on the basis of the identification by the experts, the union network is trained. Following training, the union network is able, starting from the superposition of the results identified by the two networks, that is, the portions of the images that have been considered relevant to carry out an analysis by the third network, to discriminate between an infectious state and a non-infectious state.

Algorithm 2

The second algorithm receives as input the state of the infectious state obtained by means of the first algorithm and produces as output the dosage for said infectious state. This is achieved by comparing the infectious state input with a library of known cases of infectious states, and in particular, how these states have been treated and what outcome such treatment has had on the infectious state.

Given the presence of a comparison phase, the infectious state is described in terms of "patient-related data" and "wound-related data"; these data are listed below, with the type of value of the data indicated in parentheses:

- Patient-related data:

  ○ Age (integer/float)
  ○ Sex (string)
  ○ Recent pathological anamnesis (string)
  ○ Remote pathological anamnesis (string)
  ○ Allergies (string)
  ○ Body mass index (integer/float)

- Wound-related data:

  ○ Granulation state (integer/float)
  ○ Aetiology (string; string data will be catalogued so as to be transformed into numerical data)
  ○ Area (integer/float)
  ○ Depth (integer/float)
  ○ Presence of infection (integer/float)
  ○ Body part (string)
  ○ Pain perceived by the patient (integer/float)
  ○ Applied medications (string)

It should be underlined that the data indicated above refer to the minimum amount of information necessary for algorithm 2; however, it will be evident that further data may be provided, adding additional information regarding the patient and the wound and consequently refining the precision of the inference on the dosage of the analysed infectious state.

The operation of algorithm two is now described:

1. Translation of data into temporal signals

- Each piece of data collected for a patient (reference is made to the list of data described above) is transformed into a signal that varies over time (each data point is associated with a temporal value and, through the collection over time of the specific data point, a trend over time is obtained and therefore a data point that varies over time).
- Said signals are read by the network of algorithm 2 composed of computational nodes, which monitor the trend of the signal over time.

2. Storage and comparison of clinical cases

- The network is based on a library of previous known cases, so as to be able to compare the new case with past ones.
- Optionally, the new case is stored in order to be used in the future.

3. Convolution operation

- The network performs a convolution between the data of the known cases and the case provided as input, where by convolution it is meant to compare the signals of the new case with those of the past cases in order to see how similar they are; this is achieved through a direct numerical comparison.
- If the similarity between the signals exceeds a certain threshold value, the cases are considered similar. Said threshold value is determined by the network during the training phase.

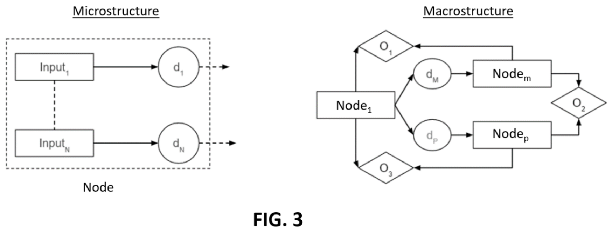

The architecture of algorithm two shown in Figure 3 is now described:

1. Micro-structure of the network

- Each signal (input) is analysed by a node that calculates the derivative of the signal, that is, the variation of the signal over time.
- The derivative is then divided into two components:

  ○ Node dp: records negative variations, for example if the wound worsens (increase in the area of the wound).
  ○ Node dm: records positive variations, for example if the wound improves (reduction in the area of the wound).

- This process is carried out for all the signals of the similar clinical cases.

2. Macro-structure of the network:

- The samples collected by the dp and dm nodes are sent to other nodes (referred to as NodeP and NodeM), which have a structure similar to the micro-structure described above.

- In the computing unit in which the network resides, the derivative is calculated again and the obtained data are compared in node O2 with a new convolution.

3. Calculation of minima and weighting of the results

- The points at which the samples differ the most from each other are identified, corresponding to the minima of the convolution, in order to understand which data most affect the worsening or improvement of the wound.

- Each result, that is, the data corresponding to the minima of the convolution, is then weighted, that is, a value is assigned indicating how much such data point positively or negatively affects the evolution of the wound.

4. Final output: Suggested dosage

- Finally, the results of the convolution are sent to nodes O3 (for deteriorations) and O1 (for improvements), together with the data relating to the dosage used.
- The system provides as output the dosage that has had the most positive or negative effects on the analysed cases, allowing identification of the most effective or harmful treatments.

In summary, algorithm two analyses patient data over time, compares known cases using convolution operations, evaluates positive and negative variations in the progression of the infectious state and finally identifies the treatments that have had the greatest positive outcome on the progression of the infectious state.

Validation

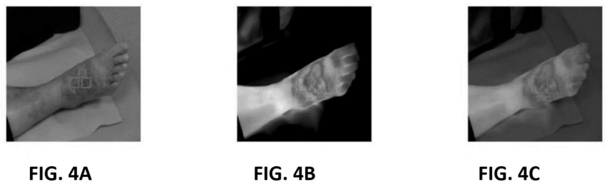

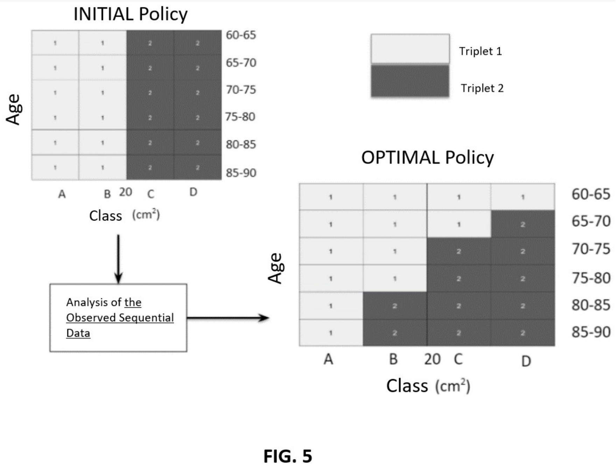

Both algorithm 1 and algorithm two were validated using public data archives. In particular, for algorithm 1, 270 pairs of RGB and thermographic images were collected and algorithm 1 was trained on them. The training was carried out as described previously. Figure 4a shows the recognition of the non-granular areas by the RGB network, Figure 4b shows the recognition of the areas with a high temperature gradient by the thermographic network, and finally Figure 4c shows the identification of the infectious state. Algorithm 2 was validated using data obtained from a clinical trial conducted in 2018. With reference to Figure 5, a group of 1000 patients was examined, all between 60 and 90 years of age, affected by ulcers of the lower limbs. During the study, the physician recorded the parameters described in the section relating to algorithm 2.

The ulcers were classified into four categories based on their extent:

- State A: from 0 to 10 cm$^2$
- State B: from 10 to 20 cm$^2$
- State C: from 20 to 30 cm$^2$
- State D: from 30 to 40 cm$^2$

Based on the state of the wound, two types of treatment were applied:

- T1: triple medication 1
- T2: triple medication 2

The data were collected in a matrix in which the columns are defined by the ulcer state and the rows by the patient age range; in particular, the age ranges are: 60-65, 65-70, 70-75, 75-80, 80-85, 85-90.

In this validation experiment, the starting point was an initial policy (see Figure 5); subsequently, the data contained in the matrix were provided to algorithm 2, and as output a matrix was obtained in which each element of the matrix was assigned the optimal T1 or T2.

It should be underlined that the number of treatments is not necessarily limited to two, but may be extended to N, where N are the possible treatments.

**Claims**

1. Computer-implemented method for identifying and treating infectious states in a patient's wound, comprising:

    • Receiving a first image, RGB, of the wound captured using an RGB camera,
    • Receiving a second image, thermographic, of the same wound captured using a thermal imaging camera, aligned with the camera, such that the second image shares a common reference system with the first image,
    • Processing the first image with first identification means to identify a wound contour based on an analysis of a texture variation in the first image,
    • Processing the second image with second identification means to identify regions with a temperature gradient exceeding a predetermined threshold,
    • Selection of regions falling in or on the contour,
    • Processing said selected regions, by third identification means configured to compare said regions with images classified according to a respective value of a parameter indicating an infectious stage;
    • Acquisition of patient characteristic data;
    • Composition of an input data set containing said value of said parameter and said characteristic data of the patient;
    • Selection of an output data set from a plurality of data sets previously stored in a database, by means of a likelihood algorithm, wherein a respective therapy is associated with each of said previously stored data sets;
    • Indication of the therapy associated with the output data set.

2. Method according to claim 1, wherein the first identification means include a software module implementing a statistical algorithm based on the comparison of textures contained in RGB images.

3. Method according to claims 1-2, wherein the second identification means include a software module implementing a statistical algorithm based on the comparison of temperature gradients contained in thermographic images.

4. Method according to the preceding claims wherein, the statistical algorithm of the first and second means includes a supervised neural network.

5. A computer-implemented method according to any preceding claim, wherein the first identification means is a CNN configured to process RGB images, the second means is a CNN configured to process thermographic images, and the third means is a CNN configured to process the output data from said first and second algorithms.

6. A computer-implemented method according to any preceding claim, wherein the likelihood algorithm is a supervised machine learning algorithm configured to perform comparisons with a data set from the database.

7. A computer-implemented method according to claim 1, wherein the data set and wound characteristics are at least:

    • Patient data:

        ◦ Age (integer/float),
        ◦ Gender (string),
        ◦ Recent medical history (string),
        ◦ Past medical history (string),
        ◦ Allergies (string),
        ◦ Body mass index (integer/float),

    • Wound data:

        ◦ Granulation state (integer/float),
        ◦ Etiology (string),
        ◦ Area (integer/float),
        ◦ Depth (integer/float),
        ◦ Presence of infection (integer/float)
        ◦ Body part (string),
        ◦ Pain perceived by the patient (integer/float),
        ◦ Dressings applied (string),

    wherein the string data are converted to numbers preferably using encoding techniques, such as ordered labeling or one-hot encoding.

8. Device for identifying and treating infectious states in a patient's wound, comprising:

• An RGB camera for capturing a first image of the wound,
• a thermal imaging camera, for capturing a second, thermographic, image of the same wound as the first image, aligned with the RGB camera so that the second image shares the same reference system with the first image,
• A processing unit connected in data exchange with the RGB camera and the thermal imaging camera and programmed to:

◦ Process the first image with first identification means to identify a wound contour based on an analysis of a texture variation in the first image,
◦ Process the second image with second identification means to identify regions with a temperature gradient exceeding a predetermined threshold,
◦ Select the regions falling in or on the contour,
◦ Process said selected regions, by means of third identification means configured to compare said regions with images classified according to a respective value of a parameter indicating an infectious stage;
◦ Acquire characteristic data of the patient;
◦ Composing an input data set containing said value of said parameter and said characteristic data of the patient;
◦ Selecting an output data set from a plurality of data sets previously stored in a database, by means of a likelihood algorithm, wherein a respective therapy is associated with each of said previously stored data sets;
◦ Indicating the therapy associated with the output data set.

9. Device according to claim 8, wherein the RGB camera comprises CMOS sensors or CCD sensors.

10. Device according to claims 8 and 9, wherein the minimum resolution of the RGB camera is 5 Mpx.

11. Device according to claim 8, wherein the minimum resolution of the thermal imaging camera is 30 px x 40 px.

12. Device according to claim 8, further comprising a display unit, preferably a monitor on which the therapy is displayed.

**FIG. 1**

**FIG. 2**

Microstructure

Macrostructure

FIG. 3

FIG. 4A

FIG. 4B

FIG. 4C

**FIG. 5**

**EUROPEAN SEARCH REPORT**

Application Number

EP 26 15 4338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 646 340 B1 (TORINO POLITECNICO [IT]) 3 January 2024 (2024-01-03) * The whole document, in particular: Paragraph [0033]; Figures 1 - 3; Claims 1, 3. * | 1-12 | INV.<br>G16H30/40<br>A61B5/00<br>G06F18/20<br>G06N3/02<br>G06T7/00 |
| Y | Becchi Sara: "Neuromorphic methods for detection, classification and analysis of chronic wounds", , 8 October 2024 (2024-10-08), pages 1-73, XP093300787, https://web.archive.org/web/20241008115106 /https://webthesis.biblio.polito.it/32093/ Retrieved from the Internet: URL:http://webthesis.biblio.polito.it/id/e print/32093 * The whole document, in particular: Pages 18 - 23, 33, 35, 40 - 46, 52, 53; Figure 4.30. * | 1-12 | G16H40/63<br>G16H50/20<br>G16H50/70 |

TECHNICAL FIELDS
SEARCHED (IPC)

G16H
A61B
G06T
G06N
G06V
G06F

The present search report has been drawn up for all claims

| Place of search | Date of completion of the search | Examiner |
|---|---|---|
| Munich | 3 June 2026 | Ruschke, Stefan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.....................................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 26 15 4338

This annex lists the patent family members relating to the patent documents cited in the above-mentioned European search report.
The members are as contained in the European Patent Office EDP file on
The European Patent Office is in no way liable for these particulars which are merely given for the purpose of information.

03-06-2026

| Patent document cited in search report | Publication date | Patent family member(s) | Publication date |
|---|---|---|---|
| EP 3646340 B1 | 03-01-2024 | EP 3646340 A1 | 06-05-2020 |
| | | IL 271933 A | 27-02-2020 |
| | | JP 7319602 B2 | 02-08-2023 |
| | | JP 2020528587 A | 24-09-2020 |
| | | US 2021358116 A1 | 18-11-2021 |
| | | WO 2019021085 A1 | 31-01-2019 |

EPO FORM P0459

For more details about this annex : see Official Journal of the European Patent Office, No. 12/82